# EUROPEAN PATENT SPECIFICATION

(11) **EP 2 081 480 B1**
(45) Date of publication and mention of the grant of the patent: **24.07.2013**
(21) Application number: 07806664.4
(22) Date of filing: 04.09.2007
(51) Int. Cl.: A61B 1/00, G02B 23/24

(54) **CAPSULE-TYPE MEDICAL DEVICE**
MEDIZINPRODUKT VOM KAPSELTYP
DISPOSITIF MEDICAL DE TYPE CAPSULE

(30) Priority: 17.11.2006 JP 2006312054
(43) Date of publication of application: 29.07.2009
(73) Proprietor: Olympus Corporation, Shibuya-ku Tokyo 151-0072 (JP)
(72) Inventor: MINAI, Tetsuo, Tokyo 151-0072 (JP); TAMURA, Kazuaki, Tokyo 151-0072 (JP); OHARA, Jin, Tokyo 151-0072 (JP)
(74) Representative: Winter, Brandl, Fürniss, Hübner, Röss, Kaiser, Polte - Partnerschaft
(86) International application number: PCT/JP2007/067209
(87) International publication number: WO 2008/059651

(56) References cited:
- WO-A1-2004/068748
- WO-A1-2006/051817
- JP-A- 2003 520 648
- JP-A- 2004 536 648
- JP-A- 2006 513 001
- JP-A- 2006 513 670
- US-A1- 2006 173 265

## Description

### Technical Field

The present invention relates to a capsule medical apparatus, and particularly to a capsule medical apparatus that is disposed in a test subject and acquires information on the inside of the test subject.

### Background Art

Endoscopes have been widely used in a medical field and the like. In particular, endoscopes in a medical field are primarily used to observe the inside of living organisms. One type of the endoscopes described above that has been proposed in recent years is a capsule endoscope that is swallowed by a test subject so that the capsule endoscope is disposed in a body cavity, the capsule endoscope capable of picking up images of subjects while moving along the body cavity through peristaltic movement, and transmitting the picked-up images of the subjects to the outside as a picked-up image signal.

An exemplary apparatus having the substantially same function as that of the capsule endoscope described above is the one proposed in Japanese Patent Application Laid-Open Publication No. 2006-513670.

Japanese Patent Application Laid-Open Publication No. 2006-513670 discloses a configuration in which an electric signal generated in a capsule endoscope disposed in a test subject is outputted to a receiver provided external to the test subject via the test subject as a conductor.

The capsule endoscope disclosed in Japanese Patent Application Laid-Open Publication No. 2006-513670 includes a first transmission electrode to which a relatively high potential is applied and a second transmission electrode to which a relatively low potential is applied, both electrode provided on the surface of a housing of the capsule endoscope. In the configuration, an electric current outputted from the first transmission electrode of the capsule endoscope disposed in the test subject flows along the surface of the test subject, and then sinks into the second transmission electrode. The receiver provided on the surface of the test subject can receive an electric signal according to the electric current outputted from the capsule endoscope based on the voltage induced between reception electrodes.

However, in the capsule endoscope disclosed in Japanese Patent Application Laid-Open Publication No. 2006-513670, a potential difference is always induced between the first and second transmission electrodes, for example, even when various electric signals, such as an picked-up image signal, are not transmitted, so that an electric current disadvantageously flows between the first and second transmission electrodes via the test subject. As a result, the remaining capacity in a built-in power supply is unnecessarily reduced.

The present invention has been made in view of the above respects and aims to provide a capsule medical apparatus capable of transmitting a signal without unnecessary reduction in remaining capacity in the built-in power supply.

WO 2004/068 748 A1 discloses a capsule according to the preamble of claim 1.

### Disclosure of Invention

### Means for Solving the Problem

A capsule medical apparatus in the present invention that is disposed in a test subject and includes an information acquisition section that acquires information on the inside of the test subject comprises the features given in claim 1.

In a preferred embodiment of the capsule medical apparatus in the present invention, the signal switching control section further controls the signal output switching section based on the output state of the electric signal to output a signal for setting the potential at each of the communication electrodes to a predetermined potential during the period in which the electric signal is not outputted.

In a preferred embodiment of the capsule medical apparatus in the present invention, the predetermined potential is the ground potential.

### Brief Description of the Drawings

Fig. 1 shows an example of the configuration of the main portion of a test subject information acquisition system using the capsule medical apparatus of the present embodiment;
Fig. 2 is a block diagram showing an example of the internal configuration of the capsule medical apparatus in Fig. 1 helpful for understanding the invention;
Fig. 3 shows an example of the detailed configuration of a drive circuit of the capsule medical apparatus in Fig. 2;
Fig. 4 is a time chart showing an example of the state of the operation of the capsule medical apparatus including the drive circuit in Fig. 3;
Fig. 5 shows an example, different from that in Fig. 3, of the detailed configuration of the drive circuit of the inventive capsule medical apparatus; and
Fig. 6 is a time chart showing an example of the state of the operation of the capsule medical apparatus including the drive circuit in Fig. 5.

### Best Mode for Carrying Out the Invention

Embodiments of the present invention and examples helpful for understanding the invention will be described below with reference to the drawings.

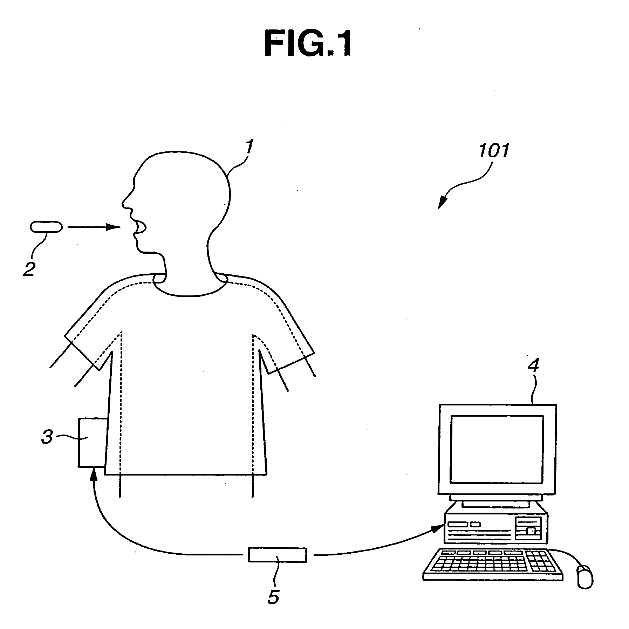

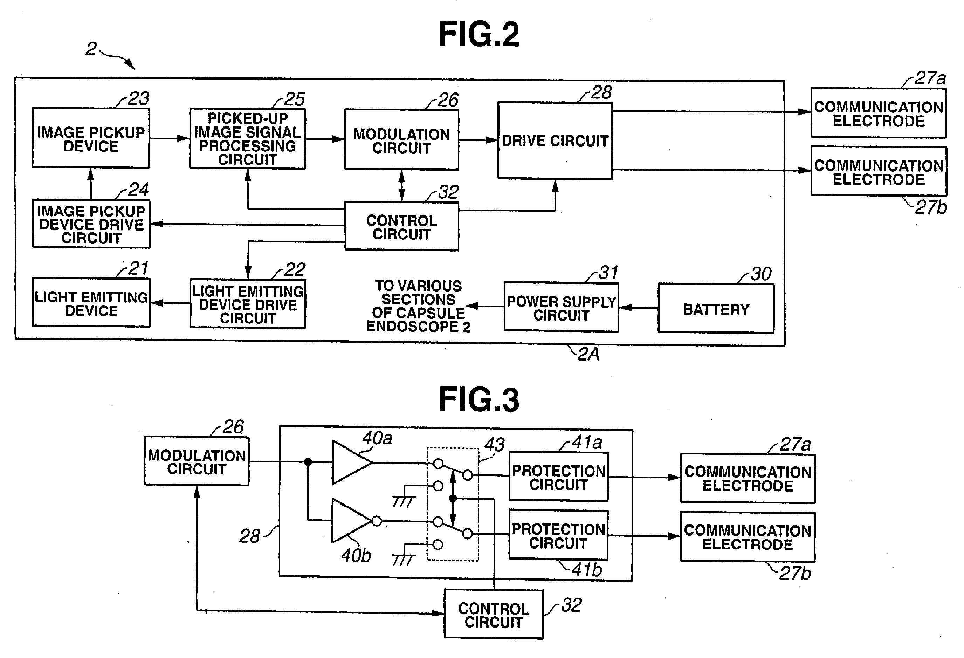

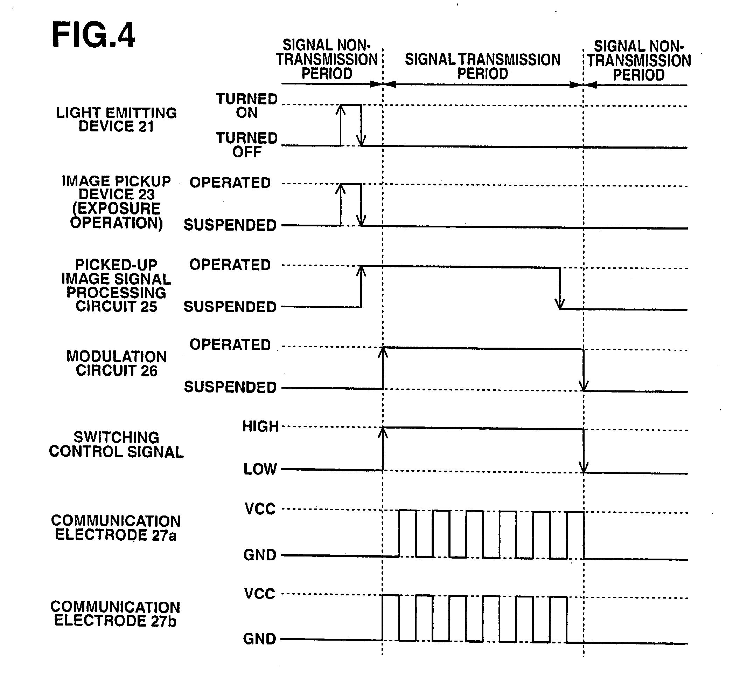

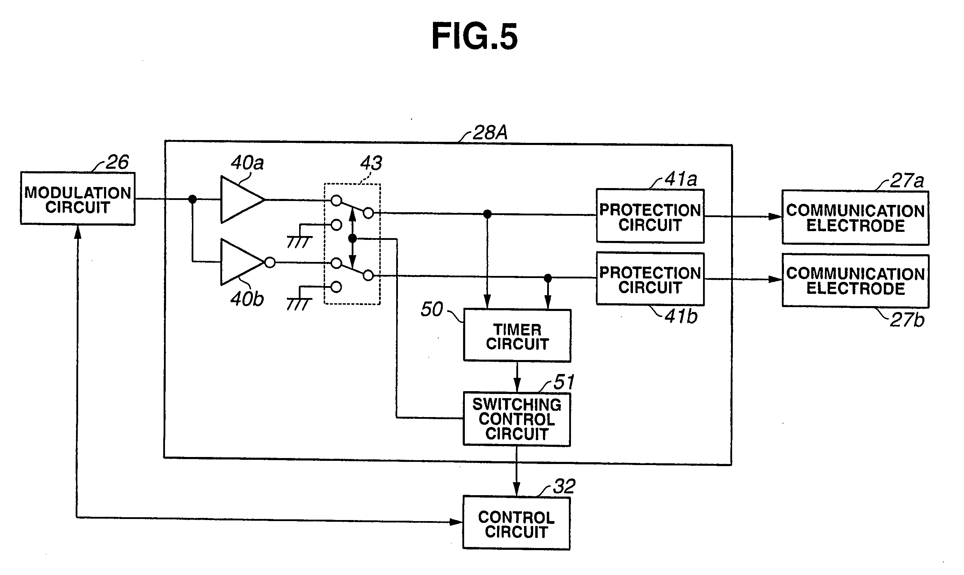

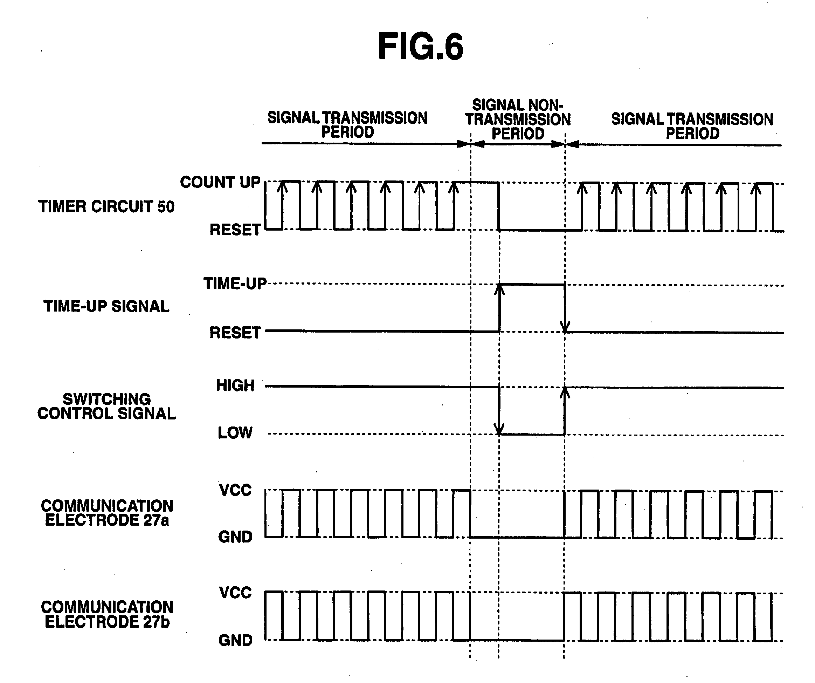

Fig. 1 shows an example of the configuration of the main portion of a test subject information acquisition system using the capsule medical apparatus of the present embodiment. Fig. 2 is a block diagram showing an example of the internal configuration of the capsule medical apparatus in Fig. 1. Fig. 3 shows an example of the detailed configuration of a drive circuit of the capsule medical apparatus in Fig. 2. Fig. 4 is a time chart showing an example of the state of the operation of the capsule medical apparatus including the drive circuit in Fig. 3. Fig. 5 shows an example of the detailed configuration of the drive circuit of the inventive capsule medical apparatus. Fig. 6 is a time chart showing an example of the state of the operation of the capsule medical apparatus including the drive circuit in Fig. 5.

A test subject information acquisition system 101 includes, as shown in Fig. 1, a capsule medical apparatus 2 swallowed by a test subject 1 so that the capsule medical apparatus 2 is disposed in a body cavity, the capsule medical apparatus 2 picking up an image of a subject present in the body cavity, a communication apparatus 3 disposed external to the test subject 1 and capable of communicating with the capsule medical apparatus 2, a terminal apparatus 4 that performs processes based on a signal or the like received by the communication apparatus 3 and displays the image of the subject, and a mobile storage medium 5 capable of inputting, outputting and recording data and the like stored in the communication apparatus 3 and the terminal apparatus 4. These apparatuses form the main portion of the test subject information acquisition system 101. A housing 2A of the capsule medical apparatus 2 is harmless to living organisms and made of an insulating material, such as resin, which does not conduct electricity from the outside.

The capsule medical apparatus 2 as the capsule medical apparatus includes, as shown in Fig. 2, a light emitting device 21 that is formed of, for example, an LED and emits illumination light for illuminating a subject, a light emitting device drive circuit 22 that controls the state of the driven light emitting device 21, and an image pickup device 23 as an information acquisition section formed of, for example, a CCD (Charge Coupled Device), the image pickup device 23 picking up an image of the subject illuminated by the light emitting device 21 and outputting the image of the subject as a picked-up image signal. The capsule medical apparatus 2 further includes an image pickup device drive circuit 24 that controls the state of the driven image pickup device 23, a picked-up image signal processing circuit 25 that performs signal processing on the picked-up image signal outputted from the image pickup device 23, a modulation circuit 26 that modulates the picked-up image signal on which the picked-up image signal processing circuit 25 has performed the signal processing, and communication electrodes 27a and 27b disposed on the surface of the housing 2A of the capsule medical apparatus 2.

The capsule medical apparatus 2 further includes a drive circuit 28 that drives the communication electrodes 27a and 27b based on the picked-up image signal outputted from the modulation circuit 26, a battery 30, a power supply circuit 31 that generates a power supply voltage Vcc for operating the various sections of the capsule medical apparatus 2 based on the power stored in the battery 30, and a control circuit 32 that primarily controls the operations of the light emitting device drive circuit 22, the image pickup device drive circuit 24, the picked-up image signal processing circuit 25, the modulation circuit 26, and the drive circuit 28.

The communication electrodes 27a and 27b have corrosion resistance to digestive juice and the like and are made of a conductive material, such as SUS316L or gold, which is harmless to living organisms.

The drive circuit 28 includes, as shown in Fig. 3, buffers 40a and 40b as a signal conversion section that generate electrode drive signals for driving the communication electrodes 27a and 27b based on the picked-up image signal modulated in the modulation circuit 26 and output the generated electrode drive signals, protection circuits 41 a and 41 b for protecting the buffers 40a and 40b, and a switching circuit 43 as a signal output switching section.

The electrode drive signal generated based on the picked-up image signal inputted to the buffer 40a is outputted to the protection circuit 41 a with the phase unchanged with respect to the picked-up image signal. On the other hand, the electrode drive signal generated based on the picked-up image signal inputted to the buffer 40b is outputted to the protection circuit 41 b with the phase shifted by 180 degrees with respect to the picked-up image signal.

Each of the protection circuits 41 a and 41 b includes a resister having, for example, approximately a few hundred ohms, and/or a capacitor to protect the buffers 40a and 40b even if the communication electrodes 27a and 27b are short-circuited.

Based on a switching control signal outputted from the control circuit 32, the switching circuit 43 switches between the signals from the buffers 40a and 40b and the signals from the ground potential points (GND) and outputs the selected signals to the protection circuits 41 a and 41 b.

In the configuration described above, based on the switching control signal outputted from the control circuit 32, the drive circuit 28 selectively outputs either the electrode drive signals generated according to the picked-up image signal modulated in the modulation circuit 26 or the signals, each having the potential at the ground potential point, to the communication electrodes 27a and 27b.

The operation of the capsule medical apparatus 2 of the example helpful for understanding the invention will now be described. Fig. 4 is a time chart showing an example of the state of the operation of the capsule medical apparatus 2 including the drive circuit 28 in Fig. 3.

The control circuit 32 as the signal switching control section monitors the picked-up image signal modulated in the modulation circuit 26, and outputs a low-level switching control signal to the switching circuit 43 during a signal non-transmission period, which is the period in which no picked-up image signal is inputted to the modulation circuit 26. Based on the low-level switching control signal, the switching circuit 43 performs switching operation that allows the signals, each having the potential at the ground potential point, to be outputted to the communication electrodes 27a and 27b via the protection circuits 41 a and 41 b. The state of the operation of the drive circuit 28 is therefore set to that in the signal non-transmission period. During the signal non-transmission period, there is thus no potential difference between the communication electrodes 27a and 27b, and hence no electric current flows between the communication electrodes 27a and 27b.

Then, the control circuit 32 controls the light emitting device drive circuit 22 to turn the light emitting device 21 on, and controls the image pickup device drive circuit 24 to expose the image pickup device 23 to light. In this way, the image pickup device 23 is exposed to light in synchronization with the timing at which the light emitting device 21 is turned on, and the image pickup device 23 outputs the picked-up image signal based on the electric charge accumulated according to the image of the subject at the timing at which the exposure is completed.

Based on an instruction from the control circuit 32, the picked-up image signal processing circuit 25 starts signal processing on the picked-up image signal outputted from the image pickup device 23 at the timing at which the exposure of the image pickup device 23 is completed, and sequentially outputs the picked-up image signal, on which the signal processing has been performed, to the modulation circuit 26. The modulation circuit 26 modulates the picked-up image signal sequentially outputted from the picked-up image signal processing circuit 25, and sequentially outputs the modulated picked-up image signal to the drive circuit 28.

On the other hand, the control circuit 32 outputs a high-level switching control signal to the switching circuit 43 at the timing at which the picked-up image signal sequentially outputted from the picked-up image signal processing circuit 25 is inputted to the modulation circuit 26. The state of the operation of the drive circuit 28 therefore changes from the state in the signal non-transmission period described above to the state in a signal transmission period.

Based on the high-level switching control signal, the switching circuit 43 performs switching operation that allows electrode drive signals generated according to the picked-up image signal outputted from the modulation circuit 26 to be outputted to the communication electrodes 27a and 27b via the protection circuits 41 a and 41 b. The state of the operation of the drive circuit 28 is therefore set to that in the signal transmission period. During the signal transmission period, a potential difference is thus generated between the communication electrodes 27a and 27b, and hence an electric current flows between the communication electrodes 27a and 27b via the surface of the test subject 1, allowing communication with the communication apparatus 3 disposed on the outer surface of the test subject 1.

Then, the control circuit 32 keeps outputting the high-level switching control signal to the switching circuit 43 until all the picked-up image signals of one frame in the image pickup device 23 are outputted from the modulation circuit 26. The operation of the drive circuit 28 is thus maintained in the state in the signal transmission period.

When the control circuit 32 detects that all the picked-up image signals of one frame in the image pickup device 23 are outputted from the modulation circuit 26, the control circuit 32 outputs the low-level switching control signal to the switching circuit 43. Based on the low-level switching control signal, the switching circuit 43 performs switching operation that allows the signals, each having the potential at the ground potential point, to be outputted to the communication electrodes 27a and 27b via the protection circuits 41 a and 41 b. The state of the operation of the drive circuit 28 is therefore set to that in the signal non-transmission period.

As described above, in the capsule medical apparatus 2 of the example, an electric current flows between the communication electrodes 27a and 27b only during the signal transmission period, while no electric current flows between the communication electrodes 27a and 27b during the signal non-transmission period. As a result, the capsule medical apparatus 2 of the example can transmit a signal without unnecessary reduction in remaining capacity in the built-in power supply.

To provide an advantageous effect substantially similar to that described above, the capsule medical apparatus 2 of the present embodiment has the drive circuit 28A in Fig. 5 instead of the drive circuit 28 in Fig. 3.

The drive circuit 28A includes a timer circuit 50 and a switching control circuit 51 as well as the sections in the drive circuit 28.

When the timer circuit 50 detects that the state of the signal outputted from the switching circuit 43 has not varied for a period greater than or equal to a predetermined period, the timer circuit 50 generates and outputs a time-up signal (indicating that time is up) to the switching control circuit 51.

Based on the time-up signal outputted from the timer circuit 50, the switching control circuit 51 outputs a switching control signal to the switching circuit 43, and also outputs, to the control circuit 32, a timing signal for showing the timing at which the switching control signal is outputted. Based on the switching control signal outputted from the switching control circuit 51, the switching circuit 43 sets the state of the operation of the drive circuit 28A to either the state in the signal transmission period or the state in the signal non-transmission period.

The operation of the capsule medical apparatus 2 including the drive circuit 28A will now be described. Fig. 6 is a time chart showing an example of the state of the operation of the capsule medical apparatus 2 including the drive circuit 28A in Fig. 5.

Based on the state of the signal outputted from the switching circuit 43, the timer circuit 50, for example, judges the period in which the signal outputted from the switching circuit 43 intermittently varies to be the signal transmission period, and maintains the state in which the time-up signal is not generated (the state in which the time-up signal is reset). The switching control circuit 51 keeps outputting the high-level switching control signal to the switching circuit 43 unless the timer circuit 50 outputs the time-up signal. Based on the high-level switching control signal, the switching circuit 43 performs switching operation that allows the electrode drive signals generated according to the picked-up image signal outputted from the modulation circuit 26 to be outputted to the communication electrodes 27a and 27b via the protection circuits 41 a and 41 b. The state of the operation of the drive circuit 28A is therefore set to that in the signal transmission period. During the signal transmission period, a potential difference is thus generated between the communication electrodes 27a and 27b, and hence an electric current flows between the communication electrodes 27a and 27b via the surface of the test subject 1, allowing communication with the communication apparatus 3 disposed on the outer surface of the test subject 1.

When the timer circuit 50 detects, based on the state of the signal outputted from the switching circuit 43, that the signal outputted from the switching circuit 43 has not varied for a period greater than or equal to a predetermined period, the timer circuit 50 judges that the current period is the signal non-transmission period, and generates and outputs the time-up signal to the switching control circuit 51.

The switching control circuit 51 outputs the low-level switching control signal to the switching circuit 43 at the timing at which the time-up signal is inputted from the timer circuit 50. Based on the low-level switching control signal, the switching circuit 43 performs switching operation that allows the signals, each having the potential at the ground potential point, to be outputted to the communication electrodes 27a and 27b via the protection circuits 41 a and 41 b. The state of the operation of the drive circuit 28A is therefore set to that in the signal non-transmission period. During the signal non-transmission period, there is thus no potential difference between the communication electrodes 27a and 27b, and hence no electric current flows between the communication electrodes 27a and 27b.

On the other hand, the control circuit 32 keeps monitoring the state of the modulation circuit 26 during the signal non-transmission period. When the control circuit 32 detects that the modulation circuit 26 resumes outputting picked-up image signals, the control circuit 32 controls the timer circuit 50 to stop time-up signal generation (reset the time-up signal). The switching control circuit 51 thus outputs the high-level switching control signal, and the state of the operation of the drive circuit 28A is set to that in the signal transmission period. Then, the communication with the communication apparatus 3 disposed on the outer surface of the test subject 1 is resumed.

The capsule medical apparatus 2 of the present embodiment is not limited to the one that communicates with the communication apparatus 3 disposed on the outer surface of the test subject 1 by conducting an electric current along the surface of the test subject 1, but may be the one that communicates with the communication apparatus 3 by inducing an electric field or a magnetic field oriented to the surface of the test subject 1.

The present invention is not limited to the above embodiments, but various changes and applications are of course possible to the extent that these changes and applications do not depart from the scope of the claimed invention.

The present application is filed with Japanese Patent Application No. 2006-312054 filed to Japan on November 1, 2006 as a basis for claiming priority.

## Claims

1. A capsule medical apparatus (2) that is disposed in a test subject (1) and includes an information acquisition section (23) that acquires information on the inside of the test subject (1), the capsule medical apparatus (2) comprising:
a plurality of communication electrodes (27a, 27b) that are disposed on the surface of the capsule medical apparatus (2) and output the information acquired by the information acquisition section to the outside of the test subject (1);
a signal conversion section (28A) that converts an electric signal outputted according to the information acquired by the information acquisition section (23) into electrode drive signals for driving the communication electrodes (27a, 27b) by inducing a potential difference therebetween and outputs the converted signals; and
a signal output switching section (43) that selectively switches between output states of the electrode drive signals;
**characterized in that**
the signal conversion section (28A) includes a timer circuit (50) and a signal switching control section (51) that controls the signal output switching section (43) based on the output state of the electric signal to stop outputting the electrode drive signals during the period in which the electric signal is not outputted, and **in that** said timer circuit (50) generates and outputs a time-up signal to the signal switching control section (51) when the timer circuit (50) detects that the state of the signal outputted from the signal output switching section (43) has not varied for a period greater than or equal to a predetermined period.

2. The capsule medical apparatus (2) according to claim 1, wherein the signal switching control section (51) further controls the signal output switching section (43) based on the output state of the electric signal to output a signal for setting the potential at each of the communication electrodes (27a, 27b) to a predetermined potential during the period in which the electric signal is not outputted.

3. The capsule medical apparatus (2) according to claim 2, wherein the predetermined potential is the ground potential.

## Patentansprüche

1. Medizinprodukt (2) vom Kapseltyp, das in ein Testsubjekt (1) abgegeben ist und einen Informationsaufnahmeabschnitt (23) umfasst, der Information über das Innere des Testsubjekts (2) aufnimmt, wobei das Medizinprodukt (2) vom Kapseltyp Folgendes umfasst:
eine Vielzahl von Kommunikationselektroden (27a, 27b), die auf der Oberfläche des Medizinprodukts (2) vom Kapseltyp angeordnet sind und die Information, die vom Informationsaufnahmeabschnitt aufgenommen wird, zur Außenseite des Testsubjekts (1) abgeben;
einen Signalumwandlungsabschnitt (28A), der ein elektrisches Signal, das gemäß der Information ausgegeben wird, die vom Informationsaufnahmeabschnitt (23) aufgenommen wurde, in Elektrodenbetriebssignale zum Betrieb der Kommunikationselektroden (27a, 27b) umwandelt, indem er eine Potentialdifferenz dazwischen induziert und die umgewandelten Signale ausgibt; und
einen Signalausgabeumschaltabschnitt (43), der selektiv zwischen Ausgabezuständen der Elektrodenbetriebssignale umschaltet;
**dadurch gekennzeichnet, dass**
der Signalumwandlungsabschnitt (28A) eine Zeitgeberschaltung (50) und einen Signalumschaltsteuerabschnitt (51) umfasst, der den Signalausgabeumschaltabschnitt (43) auf der Grundlage des Abgabezustands des elektrischen Signals steuert, um das Ausgeben der Elektrodenbetriebssignale während des Zeitabschnitts zu stoppen, in dem das elektrische Signal nicht ausgegeben wird, und dadurch, dass die Zeitgeberschaltung (50) ein Zeitendesignal erzeugt und an den Signalumschaltsteuerabschnitt (51) ausgibt, wenn die Zeitgeberschaltung (50) erfasst, dass sich der Zustand des von dem Signalausgabeumschaltabschnitt (43) ausgegebenen Signals für eine Zeitdauer nicht verändert hat, die gleich einer oder länger als eine vorab festgelegte Zeitdauer ist.

2. Medizinprodukt (2) vom Kapseltyp nach Anspruch 1, wobei der Signalumschaltsteuerabschnitt (51) weiterhin den Signalausgabeumschaltabschnitt (43) auf der Grundlage des Ausgabezustands des elektrischen Signals steuert, um ein Signal auszugeben, um das Potential an jeder der Kommunikationselektroden (27a, 27b) während des Zeitabschnitts, in dem das elektrische Signal nicht ausgegeben wird, auf ein vorab festgelegtes Potential einzustellen.

3. Medizinprodukt (2) vom Kapseltyp nach Anspruch 2, wobei das vorab festgelegte Potential das Erdpotential ist.

## Revendications

1. Appareil médical de type capsule (2) qui est disposé dans un sujet de test (1) et comprend une section acquisition d'informations (23) qui acquiert des informations sur l'intérieur du sujet de test (1), l'appareil médical de type capsule (2) comprenant :
une pluralité d'électrodes de communication (27a, 27b) qui sont disposées sur la surface de l'appareil médical de type capsule (2) et émettent, à l'extérieur du sujet de test (1), les informations acquises par la section acquisition d'informations ;
une section conversion du signal (28A) qui convertit un signal électrique émis selon les informations acquises par la section acquisition d'informations (23) en signaux de commande d'électrodes pour commander les électrodes de communication (27a, 27b) par induction d'une différence de potentiel entre celles-ci et émet les signaux convertis ; et
une section commutation d'émission de signal (43) qui commute sélectivement entre les états d'émission des signaux de commande des électrodes ;
**caractérisé en ce que**
la section conversion de signal (28A) comprend un circuit de minuterie (50) et une section commande de commutation de signal (51) qui commande la section commutation d'émission de signal (43) sur la base de l'état d'émission du signal électrique pour arrêter l'émission des signaux de commande d'électrodes durant la période pendant laquelle le signal électrique n'est pas émis, et **en ce que** ledit circuit de minuterie (50) génère et émet un signal de temps écoulé à la section commande de commutation de signal (51) lorsque le circuit de minuterie (50) détecte que l'état du signal émis de la section commutation d'émission de signal (43) n'a pas varié pendant une période supérieure ou égale à une période prédéterminée.

2. Appareil médical de type capsule (2) selon la revendication 1, dans lequel la section commande de commutation de signal (51) commande en outre la section commutation d'émission de signal (43) sur la base de l'état d'émission du signal électrique pour émettre un signal pour régler le potentiel au niveau de chacune des électrodes de communication (27a, 27b) à un potentiel prédéterminé durant la période pendant laquelle le signal électrique n'est pas émis.

3. Appareil médical de type capsule (2) selon la revendication 2, dans lequel le potentiel prédéterminé est le potentiel de terre.
